# EUROPÄISCHE PATENTANMELDUNG

(11) **EP 4 424 340 A1**
(43) Veröffentlichungstag der Anmeldung: **04.09.2024**
(21) Anmeldenummer: 24159405.0
(22) Anmeldetag: 23.02.2024
(51) Int. Cl.: A61M 1/14, A61M 1/16, A61M 1/36

(54) **PUMPENKALIBRIERVORRICHTUNG FÜR EINE DIALYSEMASCHINE UND KALIBRIERUNGSVERFAHREN FÜR EINE PUMPE**

(30) Priorität: 01.03.2023 DE 102023105086
(71) Anmelder: B. Braun Avitum AG, 34212 Melsungen (DE)
(72) Erfinder: JANIK, Waldemar, 34212 Melsungen (DE); LINDNER, Joshua, 34123 Kassel (DE)
(74) Vertreter: Winter, Brandl - Partnerschaft mbB

(57) **Zusammenfassung**

Die vorliegende Offenbarung betrifft eine Pumpenkalibriervorrichtung (1) für eine Dialysemaschine (25) mit einem Behälter (5), der ausgebildet ist, ein Flüssigkeitsvolumen (Vb) aufzunehmen und einen Zulauf (7) und einen Ablauf (9) hat, einem ersten Niveausensor (17), der ein erstes Flüssigkeitsniveau in dem Behälter (5) bestimmt und ein erstes Signal ausgibt, einem zweiten Niveausensor (19), der ein zweites Flüssigkeitsniveau bestimmt und ein zweites Signal ausgibt, einer Pumpe (3; 27), die ausgebildet ist, basierend auf zumindest einem Antriebsparameter die Flüssigkeit in oder aus dem Behälter (5) über den Zulauf (7) oder den Ablauf (9) zu fördern, und einer Auswerteeinheit (23), die basierend auf dem Antriebsparameter der Pumpe (3; 27), einem auf dem ersten Signal und dem zweiten Signal basierenden Zeitkennwert (Δt) und dem Flüssigkeitsvolumen (Vb) eine Förderrate (Qb) der Pumpe (3; 27) bestimmt und/ oder einen Kalibrierungsfaktor (a; b) berechnet. Die Offenbarung betrifft weiterhin ein Verfahren und eine Dialysemaschine.

## Beschreibung

### Technisches Gebiet

Die vorliegende Offenbarung betrifft eine Pumpenkalibriervorrichtung für eine Dialysemaschine, eine Dialysemaschine mit der Pumpenkalibriervorrichtung und ein Verfahren zum Kalibrieren einer Pumpe, insbesondere einer Ultrafiltrationspumpe.

### Hintergrund der Erfindung

Medizinische Pumpen in Form von Schlauchpumpen (auch als Peristaltik- oder Dosierpumpen bezeichnet), werden in der Medizin verbreitet eingesetzt, da die zu fördernde Flüssigkeit nicht mit der Pumpe oder Teilen der Pumpe in Berührung kommt. Beispielsweise finden derartige Pumpen in Dialysemaschinen Anwendung. Eine Funktionsweise von Schlauchpumpen beruht auf der Erzeugung eines Unterdrucks in einem Schlauch. Ein Pumpenkopf drückt mechanisch während einer Rotation des Pumpenkopfs mittels Rollen oder ähnlichem auf den Schlauch, wobei der Schlauch lokal komprimiert wird. Dabei entsteht in dem Schlauch ein Unterdruck, der ein zu förderndes Medium ansaugt. In Rollenzwischenräumen kann sich der Schlauch lokal dekomprimieren.

Eine Förderrate der Schlauchpumpe ist in hohem Maße von einer Konsistenz bzw. einer Elastizität eines den Schlauch ausbildenden Schlauchmaterials abhängig. Die Elastizität kann sich von Schlauch zu Schlauch, insbesondere von Schlauchcharge zu Schlauchcharge, stark unterscheiden. Weiterhin kann sich die Elastizität des Schlauchmaterials im Laufe einer Zeit, beispielsweise durch einen Medieneinfluss, UV-Strahlung oder dergleichen, verändern und ist weiterhin temperaturabhängig.

Auch andere Bauformen von Pumpen weisen eine Veränderung in der Fördererrate über die Zeit aufgrund von Alterung, Verschleiß, Veränderung von Umgebungsbedingungen und dergleichen auf.

### Stand der Technik

Aus dem Stand der Technik ist es allgemein bekannt, dass manuell bzw. mit externen, teuren Förderratenbestimmungsanlagen in regelmäßigen Abständen die Förderrate der medizinischen Pumpe durch einen Bediener überprüft wird. Dies hat einerseits den Nachteil, dass eine Veränderung der Fördererrate im Zweifelsfall nicht rechtzeitig erkannt wird und so ein Behandlungsergebnis beeinflusst und eine Patientensicherheit gefährdet wird. Andererseits ist das manuelle Bestimmen teuer und aufwendig.

### Kurzbeschreibung der Offenbarung

Aufgabe der vorliegenden Offenbarung ist es daher, die Nachteile aus dem Stand der Technik zu beheben oder zumindest zu reduzieren. Konkret ist es Aufgabe der vorliegenden Offenbarung, eine Förderrate einfacher und günstiger als im Stand der Technik zu bestimmen.

Diese Aufgabe wird gelöst durch eine Kalibriervorrichtung einer Pumpe insbesondere Ultrafiltrationspumpe nach dem unabhängigen Anspruch 1, einer Dialysemaschine mit der Pumpenkalibriervorrichtung sowie einem Verfahren zum Kalibrieren einer Pumpe nach den nebengeordneten Ansprüchen. Vorteilhafte Weiterbildungen sind Teil der Unteransprüche oder nachfolgend beschrieben.

Konkret wird die Aufgabe gelöst durch eine Kalibriervorrichtung einer Pumpe, insbesondere Ultrafiltrationspumpe für eine oder einer Dialysemaschine, wobei die Kalibriervorrichtung ausgebildet oder ausgestattet ist mit einem Behälter, der dafür vorgesehen und ausgebildet ist, ein vorbestimmtes Flüssigkeitsvolumen aufzunehmen und zumindest einen Zulauf und einen Ablauf hat. Weiterhin hat die Kalibriervorrichtung einen ersten, oberen Niveausensor, der dafür vorgesehen und ausgebildet ist, ein erstes Flüssigkeitsniveau in dem Behälter zu bestimmen und ein erstes Signal auszugeben und einen zweiten, unteren Niveausensor, der dafür vorgesehen und ausgebildet ist, ein zweites Flüssigkeitsniveau, welches niedriger ist als das erste Flüssigkeitsniveau, in dem Behälter zu bestimmen und ein zweites Signal auszugeben. Zusätzlich beinhaltet die (Pumpen-)Kalibriervorrichtung eine Pumpe, die ausgebildet ist, basierend auf zumindest einem Antriebsparameter die Flüssigkeit über den Ablauf aus dem Behälter zu fördern, und eine Auswerteeinheit, die basierend auf dem Antriebsparameter der Pumpe, einem auf dem ersten Signal und dem zweiten Signal basierenden Zeitkennwert und dem vorbestimmten Flüssigkeitsvolumen eine Förderrate der Pumpe bestimmt und/ oder einen Kalibrierungsfaktor berechnet.

In anderen Worten wird die Aufgabe gelöst durch die (Pumpen-) Kalibriervorrichtung, welche vorzugsweise in die Dialysemaschine integriert oder integrierbar ist. Die (Pumpen-)Kalibriervorrichtung beinhaltet den Behälter (zur Aufnahme einer Flüssigkeit, insbesondere einer leitfähigen Flüssigkeit), mit dem oberen Niveausensor und dem unteren Niveausensor. Der obere Niveausensor und der untere Niveausensor sind vorzugsweise in oder an einer Seitenwand des Behälters ausgebildet.

Alternativ kann sowohl der obere Niveausensor als auch der untere Niveausensor ausgehend von einer Oberseite des Behälters in bzw. an dem Behälter ausgebildet sein.

Der Behälter ist ausgebildet, das vorbestimmte Flüssigkeitsvolumen aufzunehmen, wobei das vorbestimmte Flüssigkeitsvolumen das Volumen ist, das der Behälter zwischen dem oberen Niveausensor und dem unteren Niveausensor aufnehmen kann. Sowohl der obere Niveausensor als auch der untere Niveausensor sind dafür vorgesehen und ausgebildet, ein erstes bzw. zweites Signal auszugeben, wenn ein Flüssigkeitsniveau bzw. ein Flüssigkeitsstand eine Höhe des jeweiligen Niveausensors erreicht. Die (Pumpen-Kalibriervorrichtung beinhaltet weiterhin die Pumpe, die dafür ausgebildet ist, basierend auf dem Antriebsparameter, welcher eine Antriebsgröße ist, die Flüssigkeit über den vorzugsweise in oder nahe an einem Boden des Behälters ausgebildeten Ablauf aus dem Behälter zu fördern bzw. zu pumpen. Zusätzlich beinhaltet die (Pumpen-) Kalibriervorrichtung die Auswerteeinheit / Steuereinheit/ Recheneinheit/ Speichereinheit. Die Auswerteeinheit kann basierend auf dem Antriebsparameter die Pumpe steuern. Weiterhin ist die Auswerteeinheit ausgebildet, basierend auf dem Antriebsparameter, dem Zeitkennwert und dem vorbestimmten Flüssigkeitsvolumen die Förderrate der Pumpe bestimmt und/ oder einen Kalibrierungsfaktor/ Justierungsparameter zu berechnen. Ein Berechnungsergebnis kann in der Auswerteeinheit gespeichert werden.

Durch eine derartige Ausbildung der (Pumpen-)Kalibriervorrichtung kann unter Verwendung von in der Dialysemaschine ausgebildeten Baugruppen die Pumpe zuverlässig, kostengünstig und regelmäßig kalibriert werden. Zusätzlich ist eine Automatisierung der Kalibrierung der Pumpe möglich.

Kern der Erfindung ist, die (Ultrafiltrations-)Pumpe der Dialysemaschine durch die (Pumpen-)Kalibriervorrichtung, welche in die Dialysemaschine integriert, integrierbar oder adaptiert ist, zu kalibrieren, indem für einen gegebenen Antriebsparameter der Pumpe der Zeitkennwert ermittelt wird, der wiedergibt, wie viel Zeit benötigt wird, ein bekanntes Flüssigkeitsvolumen durch die Pumpe zu fördern.

In einem ersten Aspekt kann es sich bei dem Zeitkennwert um eine Zeitdauer handeln, die von einem Erreichen des ersten Flüssigkeitsniveaus bis zu einem Erreichen des zweiten Flüssigkeitsniveaus verstreicht. Bevorzugt ist die Auswerteeinheit mit einer Zeiterfassungseinheit ausgebildet, die den Zeitkennwert basierend auf dem ersten Signal und dem zweiten Signal ermittelt.

In anderen Worten kann der Zeitkennwert angeben, wie lange es dauert, das bekannte Flüssigkeitsvolumen aus dem Behälter zu fördern. Alternativ kann der Zeitkennwert angeben, wie lange es dauert, das bekannte Flüssigkeitsvolumen in den Behälter zu fördern.

Ein derartiger Zeitkennwert kann einfach und kostengünstig mit störungsunanfälliger Sensorik und geringer Rechenleistung bestimmt werden. Auf diese Weise ist die (Pumpen-)Kalibriervorrichtung kostengünstig und robust ausbildbar.

In einem weiteren Aspekt kann es sich bei dem Behälter um einen Luftabscheider der Dialysemaschine handeln. In anderen Worten kann der Behälter ein in die Dialysemaschine integrierter oder an der Dialysemaschine ausgebildeter Luftabscheider sein, der beispielsweise ein Luftauslassventil bzw. eine Luftauslassöffnung aufweist.

Durch die Ausbildung des Behälters als Luftabscheider in oder an der Dialysemaschine kann auf einen zusätzlichen (Kalibrierungs-) Behälter in der Dialysemaschine verzichtet werden und die Dialysemaschine dadurch kompakter ausgebildet sein.

In einem weiteren Aspekt kann es sich bei dem ersten Niveausensor und dem zweiten Niveausensor um Leitfähigkeitssensoren handeln.

Weiterhin kann ein Leitfähigkeitsreferenzsensor in oder an dem Behälter ausgebildet sein.

In anderen Worten können der erste Niveausensor und der zweite Niveausensor als Sensoren ausgebildet sein, die das Vorhandensein einer leitfähigen Lösung an dem Sensor detektieren können und bei dem Vorhandensein der leitfähigen Flüssigkeit an dem Sensor ein entsprechendes Signal ausgeben. Alternativ sind auch Ultraschalldetektoren oder jede andere Art von Detektoren vorstellbar, die das Vorhandensein einer Flüssigkeit zuverlässig und genau detektieren können.

Bei einer Ausbildung des ersten Niveausensors und des zweiten Niveausensors als Leitfähigkeitssensoren kann optional der Leitfähigkeitsreferenzsensor in oder an dem Behälter, vorzugsweise in einer direkten Bodennähe des Behälters, ausgebildet sein.

Konkret kann der erste Niveausensor und der zweite Niveausensor jeweils in Kombination mit dem Leitfähigkeitsreferenzsensor eine Detektionseinheit ausbilden, die das Vorhandensein der leitfähigen Lösung zwischen dem ersten Niveausensor bzw. dem zweiten Niveausensor und dem Leitfähigkeitsreferenzsensor detektiert.

Durch eine Ausbildung des Niveausensors als Leitfähigkeitssensor kann eine zuverlässige Funktion sichergestellt werden, da Leitfähigkeitssensoren unanfällig gegen Störungen und zusätzlich kostengünstig sind.

Optional ist vorstellbar, den ersten Niveausensor und den zweiten Niveausensor jeweils redundant auszubilden. Anders ausgedrückt können optional mehr als einer, beispielsweise zwei, erste und/ oder zweite Niveausensoren ausgebildet sein.

Alternativ ist vorstellbar, einen Abstandssensor in einer Decke oder dem Boden des Behälters auszubilden, welcher einen Abstand zu einer Oberfläche der Flüssigkeit bestimmt, woraus das Flüssigkeitsvolumen in dem Behälter zu einem Messzeitpunkt ableitbar ist.

In einem weiteren Aspekt kann es sich bei der Pumpe um eine Ultrafiltrationspumpe handeln. Konkret kann es sich bei der Pumpe um die Ultrafiltrationspumpe der Dialysemaschine handeln, welche in die Dialysemaschine integriert oder adaptiert ist.

Weiterhin wird die offenbarungsgemäße Aufgabe gelöst durch eine Dialysemaschine, welche mit einer (Pumpen-)Kalibriervorrichtung nach einem der obigen Aspekte ausgebildet ist.

In anderen Worten ist die (Pumpen-)Kalibriervorrichtung in die Dialysemaschine integriert.

Weiterhin wird die offenbarungsgemäße Aufgabe gelöst durch ein Verfahren zum Kalibrieren einer Pumpe, insbesondere einer Ultrafiltrationspumpe, in einer Dialysemaschine mit den Schritten:
- Befüllen eines Behälters, insbesondere eines Luftabscheiders, mit einer Flüssigkeit über einen Zulauf des Behälters, bis ein erster oberer Niveausensor und ein zweiter unterer Niveausensor in dem Behälter die Flüssigkeit detektieren, wobei ein Flüssigkeitsvolumen zwischen dem ersten Niveausensor und dem zweiten Niveausensor einem vorbestimmten Flüssigkeitsvolumen Vb entspricht;
- Befüllen Stoppen;
- Betreiben der Pumpe, welche mit einem Ablauf des Behälters verbunden ist, mit einem konstanten Antriebsparameter, wobei die Flüssigkeit aus dem Behälter gepumpt wird;
- Starten einer Zeitmessung, sobald der erste Sensor keine Flüssigkeit mehr detektiert;
- Stoppen der Zeitmessung, sobald der zweite Sensor keine Flüssigkeit mehr detektiert;
- Ermitteln der Zeitdifferenz Δt zwischen dem Start der Zeitmessung und dem Stop der Zeitmessung;
- Ermitteln der Förderrate Qp über die Gleichung Qp= Vb/Δt.

In einem Aspekt kann in dem Schritt "Befüllen stoppen" optional ein kurzer Zeitpuffer vor dem Stoppen des Befüllens implementiert sein, um etwaiges Schwappen der Flüssigkeit oder dergleichen auszugleichen bzw. zu berücksichtigen. Anders ausgedrückt kann das Befüllen der Flüssigkeit in den Behälter (etwas) länger ausgeführt werden, als bis der erste obere Niveausensor die Flüssigkeit detektiert.

In einem weiteren Aspekt können die Schritte des Verfahrens unter einer Variation des Antriebsparameters zwischen einzelnen Durchläufen mehrfach wiederholt werden.

In anderen Worten kann das obige Verfahren mehrfach hintereinander wiederholt/ durchlaufen werden, wobei jeder Durchlauf mit einem abweichenden Antriebsparameter, welcher jedoch über den jeweiligen Durchlauf konstant bleibt, durchgeführt wird.

Die jeweils ermittelte Förderrate Qp kann als Wertepaar mit dem zugehörigen Antriebsparameter abgespeichert werden.

Durch eine mehrfache Ausführung kann eine Mehrpunktkalibrierung implementiert werden.

In einem weiteren Aspekt kann das Verfahren weiterhin den Schritt beinhalten:
- Bilden einer Regressionskennlinie basierend auf den ermittelten Förderraten in Bezug auf den zugehörigen Antriebsparameter.

In einem weiteren Aspekt kann das Verfahren den Schritt:
- Vergleich der ermittelten Förderrate Qp mit einer hinterlegten Förderrate und berechnen eines Kalibrierungsfaktors
beinhalten.

In anderen Worten kann in einem weiteren Schritt ermittelt werden, inwieweit sich die tatsächliche ermittelte Förderrate Qp der Pumpe von der hinterlegten (Soll-) Förderrate unterscheidet.

In einem weiteren Aspekt kann der Antriebsparameter eine Spannung U oder ein Tastverhältnis DC oder eine Frequenz f sein.

In einem weiteren Aspekt kann das Befüllen des Behälters mittelbar über eine Bilanzkammer der Dialysemaschine erfolgen.

In anderen Worten kann durch ein geschicktes Verschalten von in der Dialysemaschine ausgebildeten Ventilen der Behälter über Leitungen und Baugruppen, welche ohnehin Teil der Dialysemaschine sind, befüllt werden, sodass auf zusätzliche Anschlüsse und Leitungen an dem Behälter verzichtet werden kann. Dies reduziert Kosten und Bauraum der Dialysemaschine erheblich.

### Kurzbeschreibung der Figuren

Fig. 1 ist eine schematische Darstellung einer offenbarungsgemäßen Pumpenkalibriervorrichtung mit einem Behälter;
Fig. 2 ist eine erste schematische Darstellung der Pumpenkalibriervorrichtung in einem eingebauten Zustand;
Fig. 3 ist eine zweite schematische Darstellung der Pumpenkalibriervorrichtung in dem eingebauten Zustand;
Fig. 4 ist eine Darstellung eines Graphen einer Einpunktkalibrierung;
Fig. 5 ist eine Darstellung eines Graphen einer Mehrpunktkalibrierung;
Fig. 6 ist eine dritte schematische Darstellung der Pumpenkalibriervorrichtung in dem eingebauten Zustand; und
Fig. 7 zeigt einen alternativen Behälter der offenbarungsgemäßen Pumpenkalibriervorrichtung.

### Beschreibung der Ausführungsbeispiele

Nachstehend werden Ausführungsbeispiele der vorliegenden Offenbarung auf der Basis der zugehörigen Figuren beschrieben.

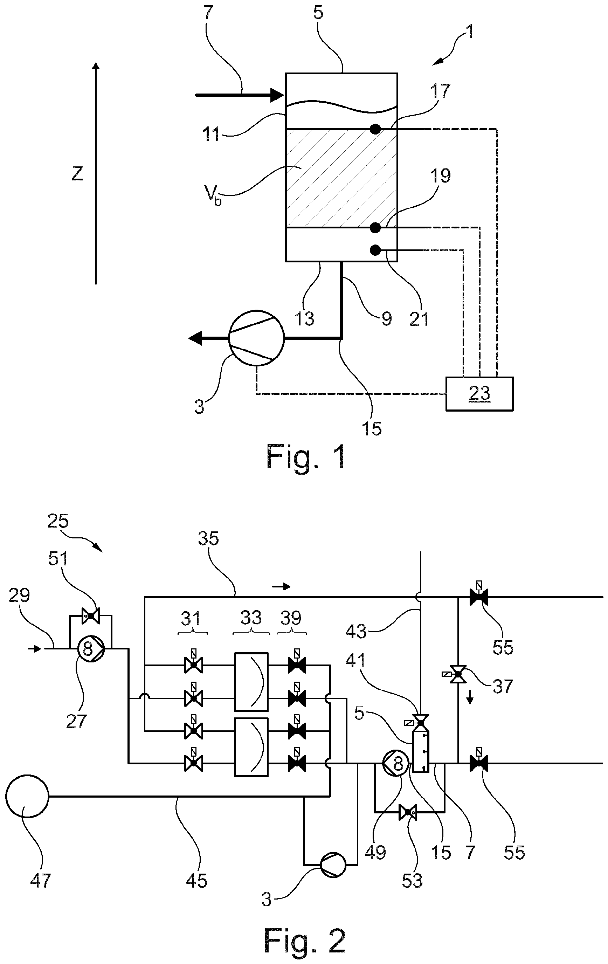

Fig. 1 zeigt eine offenbarungsgemäße Pumpenkalibriervorrichtung 1 in einer schematischen exemplarischen Darstellung, welche insbesondere einer Veranschaulichung des Funktionsprinzips dient. Die Pumpenkalibriervorrichtung 1 beinhaltet zumindest eine Pumpe 3, bei der es sich vorzugsweise um eine Ultrafiltrationspumpe handelt und einen Behälter 5, bei dem es sich vorzugsweise um einen Luftabscheider handelt. Der Behälter 5 ist vorgesehen und ausgebildet, Flüssigkeit aufzunehmen und zu speichern. Der Behälter 5 beinhaltet einen Zulauf 7 und einen Ablauf 9, wobei der Zulauf 7 in einer Höhenrichtung Z höher angeordnet ist als der Ablauf 9. Die Pumpe 3 kann auch an anderer Stelle/ Position relativ zu dem Behälter 5 ausgebildet sein, so lange die Pumpe 3 mit dem Zulauf 7 oder dem Ablauf 9 verbunden/ verbindbar ist. Über den Zulauf 7 kann die Flüssigkeit in den Behälter 5 gelangen und über den Ablauf 9 kann die Flüssigkeit den Behälter 5 verlassen. Insbesondere ist der Zulauf 7 in einer umfänglichen Seitenwand 11 des Behälters 5 ausgebildet und der Ablauf 9 ist in einem Boden 13 des Behälters 5 ausgebildet. Alternativ kann der Ablauf ebenfalls in der Seitenwand 11 des Behälters 5 ausgebildet sein. Der Ablauf 9 ist über eine Pumpenleitung 15 mit einer Saugseite der Pumpe 3 verbunden. In dem Behälter 5 sind ein oberer Niveausensor 17 und ein unterer Niveausensor 19 ausgebildet, wobei der obere Niveausensor 17 in einer Höhenrichtung Z höher angeordnet ist als der untere Niveausensor 19. Zwischen dem oberen Niveausensor 17 und dem unteren Niveausensor 19 ist ein Flüssigkeitsvolumen Vb in dem Behälter 5 aufnehmbar. Der obere Niveausensor 17 ist in der Höhenrichtung Z tiefer angeordnet als der Zulauf 7 und der untere Niveausensor 19 ist oberhalb des Bodens 13 und damit in der Höhenrichtung Z höher als der Ablauf 9 angeordnet. Selbstverständlich sind auch alternative Ausbildungen/ Anordnungen vorstellbar. So kann alternativ auch der Zulauf 7 unterhalb des oberen Niveausensors 17 ausgebildet sein. Der obere Niveausensor 17 und der untere Niveausensor 19 sind in der hier dargestellten Ausführungsform in oder an der Seitenwand 11 ausgebildet. Der Behälter 5 beinhaltet weiterhin einen Referenzsensor 21. Der Referenzsensor 21 ist hier in direkter Bodennähe des Behälters 5 ausgebildet. Es ist jedoch ausreichend, wenn der Referenzsensor 21 unterhalb von dem oberen Niveausensor 17 und dem unteren Niveausensor 19 ausgebildet ist. Der obere Niveausensor 17, der untere Niveausensor 19 und der Referenzsensor 21 sind in der hier dargestellten Ausführungsform als Leitfähigkeitssensoren ausgebildet. In der hier dargestellten Ausführungsform wird ein Füllstand über das Vorhandensein bzw. Nicht-Vorhandensein einer leitfähigen Lösung/ eines leitfähigen Mediums zwischen dem oberen Niveausensor 17 bzw. dem unteren Niveausensor 19 und dem Referenzsensor 21 ermittelt. Wenn sich die leitfähige Lösung zwischen dem oberen Niveausensor 17 bzw. dem unteren Niveausensor 19 und dem Referenzsensor 21 befindet, wird ein Stromkreis über die leitfähige Lösung geschlossen.

Alternativ ist jede Art von Sensoren vorstellbar, die einen Flüssigkeitsstand bzw. ein Vorhandensein der Flüssigkeit an einer definierten Stelle/ Höhe in dem Behälter 5 bestimmen können. Beispielsweise könnten alternativ Ultraschalldetektoren ausgebildet sein. Bei einer Ausbildung als Ultraschalldetektoren kann auf den Referenzsensor 21 optional verzichtet werden.

Weiterhin ist eine Ausbildung mit nur einem einzelnen Ultraschalldetektor vorstellbar, der ausgehend von der Oberseite des Behälters einen Abstand zu einer Flüssigkeitsoberfläche und so den Füllstand der Flüssigkeit in dem Behälter bestimmt.

Weiterhin beinhaltet die Pumpenkalibriervorrichtung 1 eine Steuer- und Auswerteeinheit 23, welche im Folgenden als die Auswerteeinheit 23 bezeichnet wird. Die Auswerteeinheit 23 ist mit der Pumpe 3, dem oberen Niveausensor 17, dem unteren Niveausensor 19 und dem Referenzsensor 21 verbunden.

Fig. 2 zeigt die Pumpenkalibrierungsvorrichtung 1 schematisch in einem eingebauten Zustand in einer Dialysemaschine 25. Es ist zu verstehen, dass lediglich ein für die vorliegende Offenbarung relevanter Ausschnitt einer exemplarischen Dialysemaschine 25 dargestellt und beschrieben wird.

Konkret zeigt Fig. 2 die Dialysemaschine 25 während eines Befüllvorgangs, in welchem der Behälter 5 mit der Flüssigkeit, insbesondere der Kalibrierflüssigkeit befüllt wird. Die Dialysemaschine 25 beinhaltet eine Vielzahl von Ventilen. Das Befüllen des Behälters 5 kann durch ein geschicktes Verschalten der Ventile ermöglicht werden.

In der hier gezeigten Ausführungsform beinhaltet die Dialysemaschine 25 eine erste Flusspumpe 27, welche aus einer ersten Leitung 29 die Flüssigkeit fördert. Die erste Flusspumpe 27 fördert die Flüssigkeit über erste Ventile 31, die hierbei geöffnet sind, in eine Bilanzkammer 33. Aus der Bilanzkammer 33 gelangt die Flüssigkeit über eine zweite Leitung 35 über ein erstes Bypassventil 37 in den Behälter 5. Da Bilanzkammerventile 39 geschlossen sind und die Pumpe 3 abdichtet, steigt ein Füllstand in dem Behälter 5 an, wobei Luft, welche sich in dem Behälter 5 befindet, über ein an dem Behälter 5 ausgebildetes Luftabscheiderventil 41 und eine Luftabscheiderleitung 43 entweichen kann.

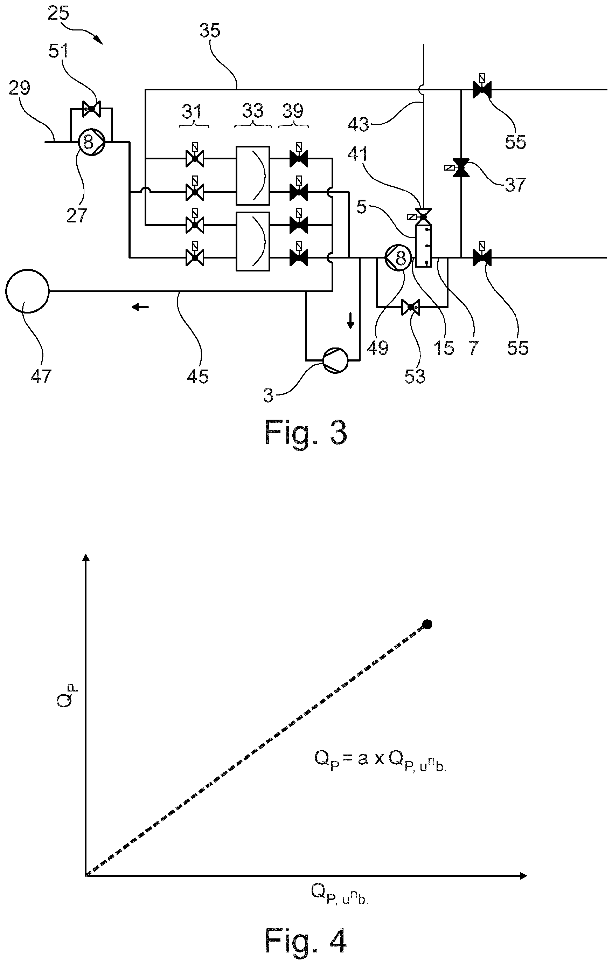

Fig. 3 zeigt die Dialysemaschine 25 während eines Kalibriervorgangs. Bei dem Kalibriervorgang steht die Flusspumpe 27 und das erste Bypassventil 37 ist geschlossen. Die zu kalibrierende Pumpe 3 fördert die Flüssigkeit aus dem Behälter 5 an der Bilanzkammer 33 vorbei durch eine dritte (Abfluss-) Leitung 45 in einen Abfluss 47. Eine zweite, an den Behälter 5 angeschlossene Flusspumpe 49 kann währenddessen stehen, da sie nicht abdichtet und somit durchlässig ist. Alternativ ist auch ein langsames Drehen/ Mitlaufen der zweiten Flusspumpe 49 denkbar, um einem hohen Unterdruck an der Saugseite der Pumpe 3 entgegenzuwirken. Da die Bilanzkammerventile 39 geschlossen sind, senkt die zweite Flusspumpe 49 nicht den Füllstand in dem Behälter 5. Die Dialysemaschine 25 beinhaltet weiter ein erstes Rückschlagventil 51 und ein zweites Rückschlagventil 53, welche ausgebildet sind, die erste Flusspumpe 27 bzw. die zweite Flusspumpe 49 zu überbrücken. Das erste Rückschlagventil 51 und das zweite Rückschlagventil 53 öffnen bei einer Überschreitung eines vorbestimmten Druckschwellenwerts, sodass die Flüssigkeit bei sich drehender erster Flusspumpe 27 bzw. zweiter Flusspumpe 49 im Bypass gefördert wird und so das System vor Beschädigung geschützt wird.

Sperrventile 55 sperren sowohl beim Befüllvorgang als auch beim Kalibriervorgang Leitungen zu einem Dialysator bzw. zu einer Spülbrücke (nicht dargestellt).

Nachfolgend wird ein Kalibrierungsvorgang, insbesondere anhand von Fig. 1, beschrieben.

Der Behälter 5 wird, beispielsweise in der in Fig. 2 beschriebenen Konfiguration, befüllt. Konkret wird der Behälter 5 in einem ersten Schritt über den Zulauf 7 mit der Flüssigkeit befüllt. Das Befüllen wird durch die Auswerteeinheit 23 gestoppt, sobald sowohl der obere Niveausensor 17 als auch der untere Niveausensor 19 die Flüssigkeit detektieren. Das Flüssigkeitsvolumen Vb zwischen dem oberen Niveausensor 17 und dem unteren Niveausensor 19 ist als ein vorgegebener, konstanter Wert in der Auswerteeinheit 23 hinterlegt.

In einem zweiten Schritt wird die Pumpe 3 mit einer unbekannten, aber konstanten Förderrate Qp,unb betrieben. Dies geschieht dadurch, dass die Pumpe 3 mit einem bestimmten Antriebsparameter, beispielsweise einer vorgegebenen Spannung U oder einem vorgegebenen Tastverhältnis DC oder einer vorgegebenen Frequenz f betrieben wird.

In einem dritten Schritt wird eine Zeitmessung gestartet, sobald der obere Niveausensor 17 keine Flüssigkeit mehr detektiert.

In einem vierten Schritt wird die Zeitmessung gestoppt, sobald der untere Niveausensor 19 keine Flüssigkeit mehr detektiert. Das Ergebnis der Zeitmessung, welche beispielsweise durch eine Zeiterfassungseinheit in der Auswerteeinheit durchgeführt wird, wird als Zeitdifferenz Δt in der Auswerteeinheit 23 gespeichert.

In einem fünften Schritt ermittelt die Auswerteeinheit 23 über eine Berechnungsvorschrift aus dem Flüssigkeitsvolumen Vb und der Zeitdifferenz Δt die Förderrate Qp=Vb/Δt. Die berechnete Förderrate Qp wird zusammen mit dem zugehörigen bestimmten Antriebsparameter in der Auswerteeinheit 23 gespeichert.

Die Schritte eins bis fünf können unter Variation des Antriebsparameters zwischen einzelnen Durchgängen wiederholt werden. In anderen Worten können die Durchgänge mit jeweils einer anderen konstanten Förderrate Qp,unb wiederholt durchgeführt werden.

Fig. 4 zeigt einen Graphen einer Einpunktkalibrierung. Eine einzelne ermittelte Förderrate Qp wird über der zugehörigen eingestellten konstanten Förderrate Qp,unb aufgetragen. Auf diese Weise kann, beispielsweise über die Gleichung Qp= a x Qp,unb, ein möglicher Justierungsparameter a bestimmt werden. Alternativ kann die einzelne ermittelte Förderrate Qp über der vorgegebenen Spannung U oder dem vorgegebenen Tastverhältnis DC oder der vorgegebenen Frequenz f aufgetragen werden und der Justierungsparameter a auf äquivalente Weise berechnet werden.

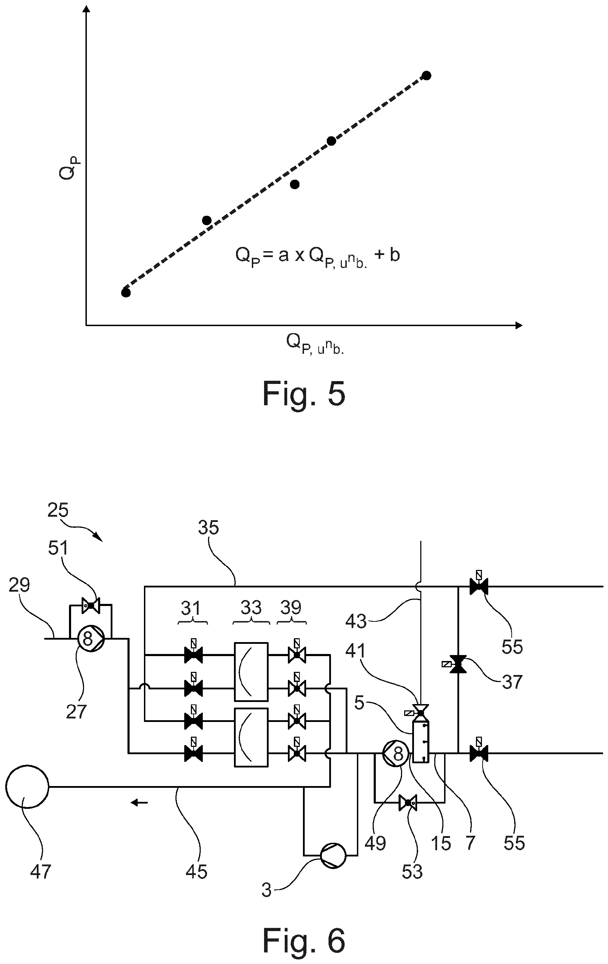

Fig. 5 zeigt einen Graphen einer Mehrpunktkalibrierung. Im Unterschied zu der Einpunktkalibrierung wird eine Anzahl von ermittelten Förderraten Qp über jeweils zugehörige eingestellte konstante Förderraten Qp,unb aufgetragen. Auf diese Weise kann, beispielsweise über die Gleichung Qp= a x Qp,unb + b, der Justierungsparameter a und ein Offset b bestimmt werden. Alternativ können die einzelnen ermittelten Förderraten Qp über der vorgegebenen Spannung U oder dem vorgegebenen Tastverhältnis DC oder der vorgegebenen Frequenz f aufgetragen werden.

Dieses Verfahren kommt bevorzugt beim automatisierten Kalibrieren und Justieren der Pumpe 3 der Dialysemaschine 25 zum Einsatz. Alternativ oder zusätzlich kann dieses Verfahren aber auch zum regelmäßigen Überprüfen der Ultrafiltrationspumpenförderrate im Rahmen eines Selbsttests eingesetzt werden.

In einer alternativen Ausführungsform, in welcher die Ventile wie in Fig. 6 geschalten sind, kann offenbarungsgemäß auch die Flusspumpe 27 kalibriert werden. Hierbei wird im Unterschied zu dem vorangehend beschriebenen Verfahren beim Befüllen die Zeitmessung gestartet, sobald der untere Niveausensor 19 die Flüssigkeit detektiert und die Zeitmessung wird gestoppt, sobald der obere Niveausensor 17 die Flüssigkeit detektiert. Dieses Verfahren ist selbstverständlich auch auf die Pumpe 3 anwendbar, wenn die Pumpe 3 in eine entgegengesetzte Richtung fördert.

In einer weiteren alternativen Ausführungsform kann auch die zweite Flusspumpe 49 kalibriert werden. Hierfür steht die Pumpe 27, die ersten Ventile 31 sind geschlossen und die Bilanzkammerventile 39 sind geöffnet. Weiterhin steht die Pumpe 3 und der Behälter ist gefüllt. Durch eine Ansteuerung der Pumpe 49 kann dann die die Pumpe 49 kalibriert werden. Hierbei ist es ausreichend, wenn nur der untere Teil der Bilanzkammer 33 auf Durchfluss geschalten ist. Dies kann realisiert werden, indem die beiden unteren der Bilanzkammerventile 39 geöffnet sind und die beiden oberen der Bilanzkammerventile 39 geschlossen sind (oder umgekehrt).

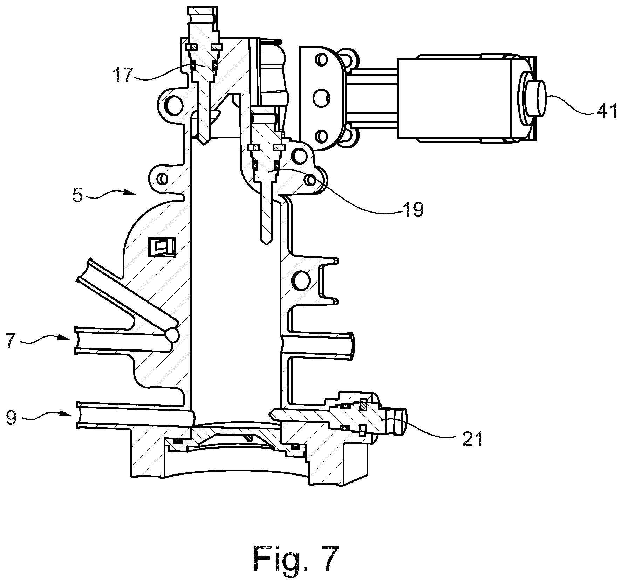

Fig. 7 zeigt eine alternative Ausführungsform des Behälters 5. Es wird nachfolgend lediglich auf Unterschiede zu dem oben beschriebenen Behälter 5 eingegangen. Sowohl der obere Niveausensor 17 als auch der untere Niveausensor 19 sind in der Oberseite des Behälters 5 ausgebildet. Der obere Niveausensor 17 ist dabei oberhalb des unteren Niveausensors 19 ausgebildet. Der Zulauf 7 und der Ablauf 9 sind unterhalb des oberen Niveausensors 17 und des unteren Niveausensors 19 ausgebildet, wobei der Zulauf 7 höher angeordnet ist als der Ablauf 9. Der Referenzsensor 21 ist in der Seitenwand ausgebildet. Weiterhin ist das Luftabscheiderventil 41 anflanschbar an den Behälter 5 ausgebildet.

### Bezugszeichenliste

- 1: Pumpenkalibriervorrichtung
- 3: Pumpe
- 5: Behälter
- 7: Zulauf
- 9: Ablauf
- 11: Seitenwand
- 13: Boden
- 15: Pumpenleitung
- 17: oberer Niveausensor
- 19: unterer Niveausensor
- 21: Referenzsensor
- 23: Auswerteeinheit/ Steuereinheit/ Recheneinheit/ Speichereinheit
- 25: Dialysemaschine
- 27: erste Flusspumpe
- 29: erste Leitung
- 31: erstes Ventil/ erste Ventile
- 33: Bilanzkammer
- 35: zweite Leitung
- 37: erstes Bypassventil
- 39: Bilanzkammerventil(e)
- 41: Luftabscheiderventil
- 43: Luftabscheiderleitung
- 45: dritte Leitung
- 47: Abfluss
- 49: zweite Flusspumpe
- 51: erstes Rückschlagventil
- 53: zweites Rückschlagventil
- 55: Sperrventil
- Z: Höhenrichtung
- Vb: Flüssigkeitsvolumen
- Qp: Förderrate
- U: Spannung
- DC: Tastverhältnis
- f: Frequenz
- a: Justierungsparameter
- b: Offset
- Δt: Zeitdifferenz

## Patentansprüche

1. Pumpenkalibriervorrichtung (1) für eine oder einer extrakorporalen Blutbehandlungsmaschine, insbesondere Dialysemaschine (25), die vorzugsweise zur Durchführung einer Ultrafiltration vorgesehen und ausgebildet ist mit
einem Behälter (5), der vorgesehen und ausgebildet ist, ein vorbestimmtes Flüssigkeitsvolumen (Vb) aufzunehmen und zumindest einen Zulauf (7) und einen Ablauf (9) hat,
einem ersten, oberen Niveausensor (17), der vorgesehen und ausgebildet ist, ein erstes Flüssigkeitsniveau in dem Behälter (5) zu bestimmen und ein erstes Signal auszugeben,
einem zweiten, unteren Niveausensor (19), der vorgesehen und ausgebildet ist, ein zweites Flüssigkeitsniveau, welches niedriger ist als das erste Flüssigkeitsniveau, in dem Behälter (5) zu bestimmen und ein zweites Signal auszugeben,
einer Pumpe (3; 27, 49), die ausgebildet ist, basierend auf zumindest einem Antriebsparameter die Flüssigkeit in oder aus dem Behälter (5) über den Zulauf (7) oder den Ablauf (9) zu fördern, und
einer Auswerteeinheit (23), die basierend auf dem Antriebsparameter der Pumpe (3; 27), einem auf dem ersten Signal und dem zweiten Signal basierenden Zeitkennwert (Δt) und dem vorbestimmten Flüssigkeitsvolumen (Vb) eine Förderrate (Qb) der Pumpe (3; 27) bestimmt und/ oder einen Kalibrierungsfaktor (a; b) berechnet.

2. Pumpenkalibriervorrichtung (1) nach Anspruch 1, wobei es sich bei dem Zeitkennwert (Δt) um eine Zeitdauer handelt, die von einem Erreichen des ersten Flüssigkeitsniveaus bis zu einem Erreichen des zweiten Flüssigkeitsniveaus verstreicht.

3. Pumpenkalibriervorrichtung (1) nach Anspruch 1 oder 2, wobei es sich bei dem Behälter (5) um einen Luftabscheider der Dialysemaschine (25) handelt.

4. Pumpenkalibriervorrichtung (1) nach einem der Ansprüche 1 bis 3, wobei es sich bei dem ersten Niveausensor (17) und dem zweiten Niveausensor (19) um Leitfähigkeitssensoren handelt und weiterhin ein Leitfähigkeitsreferenzsensor (21) in oder an dem Behälter (5) ausgebildet ist.

5. Pumpenkalibriervorrichtung (1) nach einem der Ansprüche 1 bis 4, wobei die Pumpe (3) eine Ultrafiltrationspumpe ist.

6. Extrakorporale Blutbehandlungsmaschine, insbesondere Dialysemaschine (25), die zur Durchführung einer Ultrafiltration vorgesehen und ausgebildet ist, mit einer Pumpenkalibriervorrichtung (1) nach einem der Ansprüche 1 bis 5.

7. Ultrafiltrationsmaschine die vorgesehen ist, H2O aus Patientenblut zu entziehen, mit einer semipermeablen Membran, einer Sensorik, vorzugsweise in Form einer Drucksensorik, einer Ultrafiltrationspumpe und einer Pumpenkalibriervorrichtung (1) nach einem der Ansprüche 1 bis 5.

8. Verfahren zum Kalibrieren einer Pumpe (3), insbesondere einer Ultrafiltrationspumpe, in einer Dialysemaschine (25) mit den Schritten:
- Befüllen eines Behälters (5), insbesondere eines Luftabscheiders, mit einer Flüssigkeit über einen Zulauf (7) des Behälters (5), bis ein erster oberer Niveausensor (17) und ein zweiter unterer Niveausensor (19) in dem Behälter (5) die Flüssigkeit detektieren, wobei ein Flüssigkeitsvolumen zwischen dem ersten Niveausensor (17) und dem zweiten Niveausensor (19) einem vorbestimmten Flüssigkeitsvolumen Vb entspricht;
- Befüllen stoppen;
- Betreiben der Pumpe (3), welche mit einem Ablauf (9) des Behälters (5) verbunden ist, mit einem konstanten Antriebsparameter;
- Starten einer Zeitmessung, sobald der erste Niveausensor (17) keine Flüssigkeit mehr detektiert;
- Stoppen der Zeitmessung, sobald der zweite Niveausensor (19) keine Flüssigkeit mehr detektiert;
- Ermitteln der Zeitdifferenz Δt;
- Ermitteln der Förderrate Qp über die Gleichung Qp= Vb/Δt.

9. Verfahren nach Anspruch 8, wobei die Schritte unter einer Variation des Antriebsparameters zwischen einzelnen Durchläufen mehrfach wiederholt werden.

10. Verfahren nach Anspruch 9, wobei nach der mehrfachen Wiederholung das Verfahren weiterhin den Schritt beinhaltet:
- Bilden einer Regressionskennlinie basierend auf den ermittelten Förderraten Qp in Bezug auf den zugehörigen Antriebsparameter.

11. Verfahren nach einem der Ansprüche 8 bis 10, wobei das Verfahren weiterhin den Schritt:
- Vergleich der ermittelten Förderrate Qp mit einer hinterlegten Förderrate Qh und berechnen eines Kalibrierungsfaktors (a, b)
beinhaltet.

12. Verfahren nach einem der Ansprüche 8 bis 11, wobei der Antriebsparameter eine Spannung U oder ein Tastverhältnis DC oder eine Frequenz f ist.

13. Verfahren nach einem der Ansprüche 8 bis 12, wobei das Befüllen des Behälters (5) mittelbar über eine Bilanzkammer (33) der Dialysemaschine (25) erfolgt.
